(19) Europäisches Patentamt
European Patent Office
Office européen des brevets

(11) EP 2 010 323 B1

(12) EUROPEAN PATENT SPECIFICATION

(45) Date of publication and mention
of the grant of the patent:
**14.11.2012 Bulletin 2012/46**

(51) Int Cl.:
***B01L 3/00*** (2006.01)   ***B03C 5/02*** (2006.01)

(21) Application number: **07736200.2**

(22) Date of filing: **10.04.2007**

(86) International application number:
**PCT/IL2007/000460**

(87) International publication number:
**WO 2007/116406 (18.10.2007 Gazette 2007/42)**

(54) **METHOD AND DEVICE FOR ELECTROKINETIC MANIPULATION**

VERFAHREN UND VORRICHTUNG ZUR ELEKTROKINETISCHEN HANDHABUNG

MÉTHODE ET DISPOSITIF POUR MANIPULATION ÉLECTROCINÉTIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **10.04.2006 US 790547 P**

(43) Date of publication of application:
**07.01.2009 Bulletin 2009/02**

(73) Proprietor: **Technion Research and Development of Foundation, Ltd.**
**Technion City, 32000 Haifa (IL)**

(72) Inventors:
• **GOLAN, Saar**
**34792 Haifa (IL)**
• **DINNAR, Uri**
**34759 Haifa (IL)**
• **ELATA, David**
**Palo Alto, California 94303 (US)**
• **ORENSTEIN, Meir**
**32983 Haifa (IL)**

(74) Representative: **Dorschner, David**
**Dennemeyer & Associates s.a.**
**P.O. Box 1502**
**1015 Luxembourg (LU)**

(56) References cited:
**EP-A- 1 548 103**     **EP-A- 1 764 418**
**WO-A-00/28313**      **WO-A1-2006/004558**
**US-A1- 2004 011 650**   **US-A1- 2004 055 891**
**US-A1- 2005 221 473**

**Description**

FIELD AND BACKGROUND OF THE INVENTION

The present invention relates to object manipulation and, more particularly, to a method and device for manipulating small scale objects by electrokinetics.

Electrokinetics is the use of electrical fields (and the resulting forces) to manipulate matter in a fluid medium. Electrokinetics is a term which encompasses all types of processes in which the application of electric field results in motion of matter.

One type of electrokinetics is electrophoresis. Electrophoresis is a phenomenon in which charged particles, located between two electrically biased electrodes, are influenced by the electric field generated by the electrodes such that they are attracted to one electrode and repulsed by the other electrode. The attracting and repulsing forces are proportional to the particle net charge and the electric field magnitude.

Another type of electrokinetics is dielectrophoresis. Dielectrophoresis is the motion of matter caused by polarization effects in a nonuniform electric field. Electric fields induce dielectric polarization components in polarizable particles. The extent of the particle's polarization is related to its effective dielectric constant (polarizability) and to the electric field magnitude. Particles that have high dielectric constants experience significant polarization while particles that have low dielectric constants experience lower polarization. In dielectrophoresis, particle motion is produced by the interaction between the nonuniform electric field and the dielectric polarization components induced in the particle and in the surrounding fluid medium by the field. In a uniform field, neutral particles, including neutral polarized particles, experience no net electric force. However, when placed in a nonuniform field polarizable, particles experience a net force in the direction of the field gradient, tending to move the particles towards regions of higher electric field strength. This motion is known as positive dielectrophoresis. If the polarizability of the suspension medium exceeds that of the particles, they tend to move towards regions of lower electric field strength. This motion is known as negative dielectrophoresis.

The principle of dielectrophoresis has become a popular technique for separating objects such as biological cells or microparticles in suspension. The ability to identify, characterize and purify cell subpopulations is fundamental to numerous biological and medical applications, often forming the starting point for research protocols and the basis for current and emerging clinical protocols. Cell separation has numerous applications in medicine, biotechnology and environmental study. For example, cell separation can make possible life-saving procedures such as autologous bone marrow transplantation for the remediation of advanced cancers in which the removal of cancer-causing metastatic cells from a patient's marrow is necessary. In other applications, such as the study of signaling between blood cells, highly purified cell subpopulations permit studies that would otherwise be impossible. A key advantage of dielectrophoretic separation over currently used separation techniques (such as the isolation of cells according to cell density, specific immunologic targets or receptor-ligand interactions), is that dielectrophoresis effectively maps biophysical properties into electrostatic forces whose direction and magnitude reflect cellular properties. The analysis of the dielectrophoretic motion of cells thus permits biophysical parameters, such as capacitance and surface conductance, to be probed.

Dielectrophoretic forces are generated by either conventional dielectrophoresis (cDEP, also termed classic dielectrophoresis or simply dielectrophoresis) or by traveling-wave dielectrophoresis (twDEP). Classic dielectrophoresis refers to motion arising from nonuniform distribution in the magnitude of a direct-current (DC) or alternating-current (AC) electric field. Traveling-wave dielectrophoresis refers to motion arising from nonuniform distribution in the phase of an alternating-current electric field.

The nonuniform electric fields required for the implementation of dielectrophoresis are typically generated by microelectrodes connected via electrical contacts to an AC or DC power source. Known in the art are two major techniques for generating nonuniform electric fields.

In one such techniques, the electrodes are arranged in a specialized geometry such as a castellated arrangement [Green, N. G., Morgan, H., J. Phys. D 1998, 31, L25-L30; and Morgan, H., Hughes, M. P., Green, N. G., Biophys. J. 1999, 77, 516-525]. These geometries are characterized by a variable distance between the electrodes, such that the electric field is higher in regions in which the electrodes are closer and lower in regions in which the electrodes are farther apart. This results in nonuniform electric fields.

In another techniques, the electrodes are arranged in a symmetric geometry, and the motion of particles is achieved by subjecting them to a specific voltage [Li, H., Bashir, R., Sensors aszd Actuators 2002, 86, 215-221; Talary, M. S., Burt, J. P. H., Tame, J. A., Pethig, R., J. Phys. D 1996, 29, 2198-2203; Xu, J. Q. , Wu, L., Huang, M., Yang, W., Cheng, J., Wang, X., in: Micro Total Analysis Systems, Monterey 2001, pp. 565-566; and Wang, X., Yang, J., Huang, Y., Vykoukal, J., Becker, F. F., Gascoyne, P. R. C., Anal. Chem. 2000, 72, 832-839]. The applied voltage is in the form of a pulse sequence, which is typically characterized by constant amplitude and half-cycle or quarter-cycle phase sequence. The operation of devices employing a half-cycle phase sequence is based on classic dielectrophoresis and the dielectrophoretic force produced thereby is perpendicular to the electrode plane. The operation of devices employing a quarter-cycle phase sequence is based on traveling wave dielectrophoresis and the dielectrophoretic force produced thereby is parallel to the electrode plane.

It is generally difficult to manufacture dielectrophoretic devices, *inter alia* due to the need to establish electrical contact between the microelectrodes generating the nonuniform field and the external power source. The electrodes are typically connected by metal traces to peripheral pads where electrical contacts with a signal generator are established. Attempts have been made to reduce the number of external electrical contacts in dielectrophoretic devices, by employing interdigitated electrode arrays. However, this approach may necessitate complex photolithography and repeated metal evaporation processes and is therefore costly and technologically demanding. For these reasons, dielectrophoretic devices have met with little commercial acceptance. Further prior art devices are disclosed in US-A-2004/0011650, EP-A-1548103, WO 00/28313 and WO 2006/004558.

There is thus a widely recognized need for, and it would be highly advantageous to have, a method and device for manipulating objects by electrokinetics devoid of the above limitations.

SUMMARY OF THE INTENTION

According to the present invention there is provided a device for manipulating an object present in a fluid by electrokinetics as defined in claim 1.

According to further features in preferred embodiments of the invention described below, the electrically floating electrode structure(s) is designed and configured to control non-uniformities in the electric field.

According to still further features in the described preferred embodiments the electrically floating electrode structure(s) is designed and configured to increase non-uniformities in the electric field.

According to an additional aspect there is provided a method of fabricating a device for manipulating an object by electrokinetics. The method comprises: fabricating a plurality of electrode structures in a chamber; fabricating a plurality of electrical contacts in the chamber; and connecting a portion of the plurality of electrode structures to the plurality of electrical contacts, so as to provide a plurality of electrically biasable electrode structures while maintaining and at least one electrically floating electrode structure, the electrically floating electrode structure(s) being designed and configured to increase non-uniformities in an electric field generated upon activation of the plurality of electrically biasable electrode structures.

According to still further features in the described preferred embodiments at least one of the plurality of electrically biasable electrode structures is characterized by at least one nanometric dimension. According to still further features in the described preferred embodiments the electrically biasable electrode structures are characterized by at least one micrometric dimension.

According to still further features in the described preferred embodiments the electrically floating electrode structure(s) is designed and constructed to increase a dielectrophoretic force exerted on the object by at least one, more preferably at least two, more preferably at least three orders of magnitude. According to still further features in the described preferred embodiments at least one electrically floating electrode structure is designed and constructed to increase a dielectrophoretic force exerted on the object by more than three orders of magnitude.

According to still further features in the described preferred embodiments the electrically biasable electrode structures comprise interdigitated electrodes.

According to still further features in the described preferred embodiments the electrically floating electrode structures comprise carbon nanotubes.

According to still further features in the described preferred embodiments the device or apparatus further comprises a power source device, electrically connected to the plurality of electrically biasable electrode structures and configured for applying bias thereto.

According to still further features in the described preferred embodiments the power source device is configured to provide out-of-phase signals to individual members of the plurality of electrodes.

According to still further features in the described preferred embodiments the out-of-phase signals are selected such as to generate a traveling wave dielectrophoretic force.

According to still further features in the described preferred embodiments the out-of-phase signals are selected such as to generate a classical dielectrophoretic force.

According to still further features in the described preferred embodiments the power source device is a direct-current power source device.

According to still further features in the described preferred embodiments device or apparatus further comprises a detector for detecting the presence of the object.

According to still further features in the described preferred embodiments the detector is designed and constructed for detecting variations in the electrical characteristics in a predetermined region within the chamber.

According to still further features in the described preferred embodiments the detector is designed and constructed for detecting variations in the optical characteristics in a predetermined region within the chamber.

According to still further features in the described preferred embodiments the device or apparatus further comprises at least one inlet port and at least one outlet port, the at least one inlet port and the at least one outlet port being in fluid communication with the chamber, and a fluid flow system for supplying the fluid to the at least one inlet port and removing the fluid from at least one outlet.

According to still further features in the described preferred embodiments the object is made of organic material.

According to still further features in the described preferred embodiments the object is made of non-organic material.

According to still further features in the described preferred embodiments the object comprises a biological molecule.

According to still further features in the described preferred embodiments the object comprises a non-biological molecule.

According to still further features in the described preferred embodiments the fluid is a biological fluid.

According to still further features in the described preferred embodiments the fluid is a non-biological fluid.

According to still further features in the described preferred embodiments the object is selected from the group consisting of a cell, cell aggregate, cell organelle, nucleic acid, bacterium, protozoan and virus.

According to still further features in the described preferred embodiments the object is an aggregate of inorganic matter.

According to still further features in the described preferred embodiments the object is an organic material, isomer thereof or isotope thereof.

According to still further features in the described preferred embodiments the object is suspended inorganic matter.

According to still further features in the described preferred embodiments the object is dissolved inorganic matter.

The present invention successfully addresses the shortcomings of the presently known configurations by providing a device, apparatus and method for manipulating an object by electrokinetics.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

Implementation of the present invention involves performing or completing selected tasks or steps manually, automatically, or a combination thereof. Moreover, according to actual instrumentation and equipment of preferred embodiments of the method and system of the present invention, several selected steps could be implemented by hardware or by software on any operating system of any firmware or a combination thereof. For example, as hardware, selected steps of the invention could be implemented as a chip or a circuit. As software, selected steps of the invention could be implemented as a plurality of software instructions being executed by a computer using any suitable operating system. In any case, selected steps of the method and system of the invention could be described as being performed by a data processor, such as a computing platform for executing a plurality of instructions.

BRIEF DESCRIPTION OF THE DRAWING

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

FIG. 1 is a schematic illustration of a device for manipulating an object present in a fluid, according to various of the present invention aspects, (not in accordance with claim 1)

FIG. 2a is a schematic illustration of a microfluidic device, according to various of the present invention invention aspects, (not in accordance with claim 1) ,

FIG. 2b is a schematic illustration of an apparatus for manipulating an object present in a fluid, according to various exemplary embodiments of the present invention;

FIG. 3 is a is a schematic illustration of a device invention (not in accordance with claim 1) or manipulating an object in an exemplified embodiment in which the device comprises two electrically biasable electrode structures and a electrically floating cylindrical electrode structure;

FIGs. 4a-b illustrate electric field and the normalized field intensity gradients for the device of Figure 3;

FIG. 4c shows the field intensity gradients in the vicinity of the cylindrical floating electrode structure of the device of Figure 3, as a function of the radius of the cylinder;

FIGs. 5a-d are schematic illustrations of model devices (not in accordance with claim 1) used in computer simulations;

FIGs. 6a-8h show results of computer simulations corresponding to the model devices of Figures 5a-d;

FIG. 9 shows the effect of the distance between the biased electrodes on the field intensity and field intensity gradient at the floating electrode edges, as calculated from the results of the computer simulations; and

FIGs. 10a-b are two video images captured in experiments performed using prototype devices manufactured according to various exemplary embodiments of the present invention.

## DESCRIPTION OF THE PREFERRED EMBODIMENTS

The present embodiments comprise a device and method which can be used to manipulate objects by electrokinetics. Specifically, but not exclusively, the present embodiments can be used to manipulate small scale objects by dielectrophoresis. The present embodiments further comprise a method suitable for manufacturing a device for manipulating an object.

The principles and operation of a device and method according to the present embodiments may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

While the embodiments below are described with a particular emphasis to dielectrophoretic forces, it is to be understood that a more detailed reference to dielectrophoresis is not to be interpreted as limiting the scope of the invention in any way. The device and method of the present embodiments can therefore be used to manipulate electrically neutral as well as charged objects, which can be conductive, dielectric or semiconductive. The manipulated objects can be made of any material, including, without limitation, inorganic material *e.g.*, minerals, crystals, colloidal and gas bubbles, organic material or biological material *e.g.*, cells, nucleic acids, bacteria, protozoans and viruses. The manipulated objects can also be in the form of cell aggregates, cell organelles, molecules or molecular aggregates, such as, but not limited to, proteins and nucleic acids.

While being manipulated according to various exemplary embodiments of the present invention, the objects are typically present in a fluid, such as, but not limited to, water, a biological fluid (for example, body fluid, *e.g.*, blood, plasma, urine, saliva, vaginal secretions, feces and wound excrement), a bacterial cell suspension, a protein medium, an antibody medium, a nucleic acid medium, ink and the like and fluid media commonly used in standard medical applications such as phosphate buffered saline. The fluid may include more than one type of objects, such as, but not limited to, a mixture of cell types. The device and method manipulate the objects by applying forces so as to change their kinematical properties. Depending on their various characteristics (size, mass, electrical properties, *etc.*), different types of objects may have different responses to the forces applied by the device and method of the present embodiments. Thus, the device and method of the present embodiments can be used to discriminate between distinctive types of objects because the analysis of the kinematical properties of the manipulated objects (position, velocity, acceleration) allows to identify their various characteristics or at least to separate the objects according to their different kinematical properties. For example, the present embodiments can be used to manipulate erythrocytes in a blood sample, abnormal erythrocytes (*e.g.*, erythrocyte infested with malaria) in a blood sample containing normal and abnormal erythrocytes, fetal nucleated red blood cells in a mixture of maternal blood, cancer cells in a mixture with normal cells.

The present embodiments are useful to manipulated objects of any size. In particular, the present embodiments are useful for particles in the sub-millimeter scale. A characteristic length scale for the manipulated particles can therefore be from about 1 nm to about 500 $\mu$m, more preferably from about 10 nm to about 50 $\mu$m, more preferably from about 10 nm to about 1 $\mu$m.

As used herein the term "about" refers to $\pm$ 20 %.

Small size particles can be, for example, chemical or biological molecules (including proteins, DNA, RNA, antibodies, antigens and lipids), assemblages of molecules, viruses, plasmids, bacteria, cells or cell aggregates, protozoans, embryos or other small organisms, as well as non-biological molecules, assemblages thereof, minerals, crystals, colloidal, conductive, semiconductive or dielectric particles and gas bubbles.

As demonstrated in the Examples section that follows, the device and method of the present embodiments are capable of separating cells without the need to alter them with ligands, stains, antibodies or other means. Cells remain undamaged, unaltered and viable during and following separation. Non-biological applications similarly require no such alteration. It is to be understood, however, that the device and method of the present embodiments are also suitable for separating the objects even if they have been so altered.

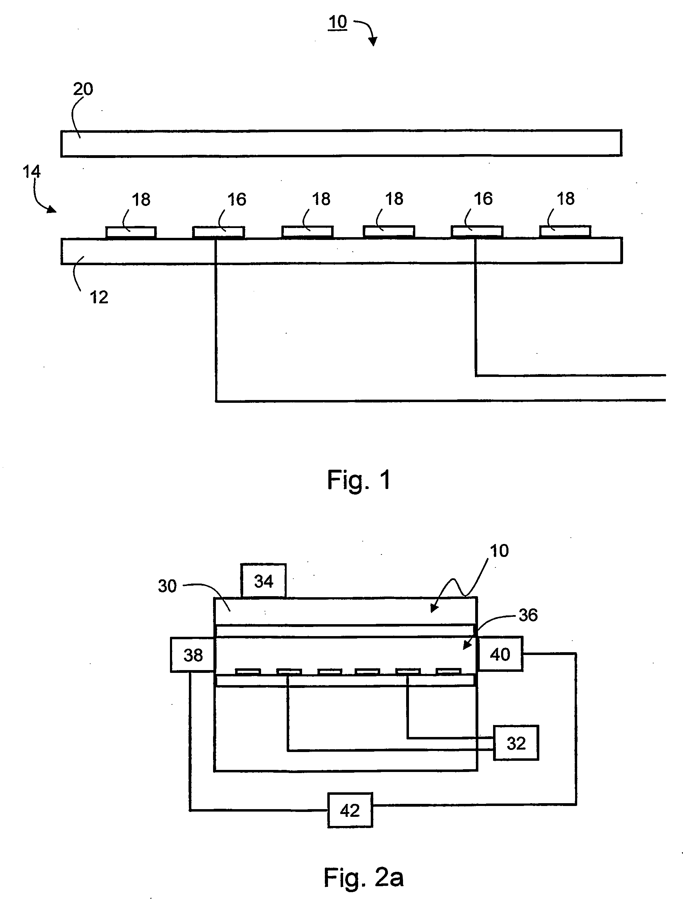

Referring now to the drawings, Figure 1 illustrates a device **10** for manipulating an object present in a fluid. Device **10** comprises a substrate **12** forming a flow chamber shown generally at **14**. Device **10** further comprises a plurality of electrically biasable electrode structures **16** and one or more electrically floating electrode structures **18**. Electrode structures **16** and **18** are formed on or integrated with substrate **12**.

As used herein, the term "floating electrode structure" refers to an electrode structure that is separated from a conductive or semiconductive body by an intervening dielectric having thickness and other properties selected to substantially prevent flow of charge carriers to the electrode structure.

As used herein, the term "biasable electrode structure" refers to an electrode structure that is configured to be electrically connected to a power source, e.g., via a contact pad or the like, in a manner such that upon activation of the power source, charge carriers flow from the power source to the electrode structure or from the electrode structure to the power source, and the electrode structure becomes electrically biased.

Optionally and preferably, device **10** comprises an additional substrate **20**, spaced apart from substrate **12** such that flow chamber **14** is defined between substrates **12** and **20**. For example, substrates **12** and **20** can be planar substrates engaging different planes and flow chamber **14** can be defined in the volume between the two different planes.

Shown in Figure 1 is a six electrode structure configuration with six parallel electrode structures such that the two outermost structures are floating electrode structures, the two next-to-outermost structures are biasable electrode structures and the two innermost structures are floating electrode structures positioned between the biasable electrode structures, device **10** can comprise any number of electrode structures in any orientation, provided there is at least one floating electrode structure and at least two biasable electrode structures. Thus, in the simplest configuration, device **10** comprises a pair of biasable electrode structures and a single floating electrode structure. In this embodiment, the floating electrode is preferably, but not obligatorily, positioned between the biasable electrodes.

Unless specifically indicated, the singular form "electrode structure" applies also to a plurality of electrode structures and vice versa.

Substrates **12** and **20** are made of electrically insulating or dielectric material which is preferably, but not obligatorily transparent to visible light to allow monitoring locomotion of objects within the device by visual or other optical means. Representative example of materials suitable for substrate **12** and **20** include, without limitation, glass, silicon dioxide, resistive (non-conductive) silicon, plastics (such as, but not limited to, those used for printed circuit board substrates), elastomers (*e.g.*, poly-dimethylsiloxane), insulating photoresists (*e.g.*, SU-8) ceramic or the like.

The electrode structures of device **10** can be made of any electrically conductive material such as, but not limited to, evaporated metal layers, conductive polymers, photoelectric materials or the like. The electrode structures may be in direct contact with the fluid or fluids, or they may be separated from them by a thin passivation layer or layers (*e.g.*, a film of $SiO_2$, a photoresist, an elastomer, an adhesive, silicon nitride or a biologically selective and functional layer).

The floating electrode structure of device **10** is designed and configured to increase and/or control nonuniformities in the electric field generated upon application of bias to the electrically biasable electrode structures. In various exemplary embodiments of the invention the floating electrode is designed and configured to provide a predetermined nonuniform electric field distribution in chamber **14**. The control and/or increment of the electric field non-uniformity can be done by selecting the number, shape, size, material and/or position of the floating electrode structure. Nonuniform electric field is particularly useful when it is desired to use device **10** for manipulating neutral objects. In this case, the nonuniform electric field exerts dielectrophoretic forces on the objects.

As demonstrated in the Examples section that follows, the floating electrode structure can significantly increase the dielectrophoretic force exerted on the object. According to a preferred embodiment of the present invention the floating electrode structure is designed and constructed to increase the dielectrophoretic force by at least one, more preferably at least two most preferably at least three orders of magnitude. In other embodiments, the dielectrophoretic force is increased by more than three orders of magnitude. Since in operation there is no bias which is applied to the floating electrode structure, the increment of the dielectrophoretic force, via the field intensity gradients, is achieved without increasing the applied bias and/or substantially increasing the electric field.

The amount by which the floating electrode structure increases the nonuniformity of the electric field in the device can be obtained experimentally, by analytic calculations and/or by numeric simulations. For example, device **10** and another device, similar to device **10** but with no floating electrode structure (or with the floating electrode structure replaced by a biasable electrode structure), can be manufactured and connected to a power source so as to generate electric field. Nonuniformities in the electric fields within the two devices can then be measured and compared to ensure that the floating electrode structure increases nonuniformities. Alternatively or additionally, the effect of the floating electrode structure can be determined by numerical simulation or theoretical analysis. Thus, the nonuniformity in the electric field for two similar devices, with and without floating electrode structure, can be calculated or numerically simulated and compared to ensure that the floating electrode structure increases nonuniformities.

The biasable and floating electrode structures can be of any size and shape. In one embodiment, the biasable and floating electrode structures are characterized by one or more micrometric dimensions. For example, the biasable and floating electrode structures can be linear electrodes having a width of from about 5 $\mu$m to about 150 $\mu$m, a length

of from about 100 μm to about 2.5 mm and a thickness of from about 10 nm to about 1 μm.

In another embodiment, the biasable electrode structures are characterized by one or more micrometric dimensions, and the floating electrode structures are characterized by one or more nanometric dimensions. For example, the biasable electrode structures can be of the shape and size described above and the floating electrode structure can be, or it can be formed of nanostructures, such as, but not limited to, carbon nanotubes, *e.g.*, fullerene carbon nanotubes which can be, either single-walled or multi walled nanotubes. This embodiment combines the advantage of micrometric biasable electrode structures from the standpoint of relatively simple manufacturing process with the advantage of nanometric floating electrode structures from the standpoint of relatively large obtainable non-uniformities in the electric field. For example, non-uniformities obtainable using a device having two linear micrometric electrode structures and two linear nanometric electrode structures are larger by approximately three orders of magnitude in comparison with nonuniformities obtainable using a device having four linear micrometric electrode structures.

Also contemplated is a configuration in which the biasable electrode structures are characterized by one or more nanometric dimensions.

The electrically biasable electrode structures can be of any shape and can be arranged in any geometrical configuration. For example, the electrically biasable electrode structures can be interdigitated electrodes.

The term "interdigitated" means that a plurality of "digits" of a first electrode group is disposed alternately with a plurality of "digits" of a second electrode group. The geometry, dimensions and overall shape of the interdigitated electrodes may vary in different embodiments.

Before providing a further detailed description of the present embodiments, attention will be given to the advantages and potential applications offered thereby.

A particular advantage of device **10** is the use of floating electrode structure, since device **10** generally includes fewer connections to external signal sources. The reduced number of connections allows miniaturization and improves conventional devices at least from the standpoint of compactness, since external signal connections tend to be bulky.

As further explained in the Examples section that follows, the dielectroplioretic force is proportional to the volume of the manipulated object. Thus, the dielectrophoretic forces experienced by small objects are significantly lower than the dielectrophoretic forces experienced by larger objects. For example, the dielectrophoretic forces experienced by nanoparticles are about $10^9$ times weaker than the dielectrophoretic forces experienced by microparticles.

One traditional approach for manipulating nanoparticles by dielectrophoresis calls for increasing the voltages applied to the electrodes. However, Function generators capable of producing such high voltages are rarely, if at all, attainable. Furthermore, working with too high voltages can be hazardous. Another traditional approach calls for the fabrication of electrodes having much smaller feature sizes and much smaller inter-electrode separation. However, for providing sufficiently high dielectrophoretic forces for the manipulation of nanoparticles, nano-electrodes may have to be fabricated. Such fabrication is known to be difficult, in particular when all the electrodes are connected to external signal sources. The device of the present embodiments successfully overcomes these difficulties because the floating electrodes do not require external signal sources.

An additional improvement presented by the device of the present embodiments is the aforementioned combination of biasable microelectrode structures and floating nanoelectrode structures. The increased dielectrophoretic force in the vicinity of a floating nanoelectrode structure of the present embodiments originates from imposing nanoscale changes on the potential distribution in these areas. As demonstrated in the Examples section that follows, although the floating nanoelectrode structures do not considerably affect the potential and the electric field values, they significantly increase the field intensity gradients. This can be explained as follows: in the vicinity of a generally cylindrical floating electrode structure, the electric field intensity does not depend on the radius of the electrode structure, but the gradient of the electric field is inversely proportional to this radius.

The gradient in the vicinity of a nanoelectrode structure is therefore about three orders of magnitude higher than in the vicinity of a microelectrode structure. Thus, in the embodiments in which the biasable electrode structures are of micrometric scale and the floating electrode structures are of nanometric scale, the electric field gradient near the floating electrode structures is significantly higher than near the biasable electrode structures. Such configuration allows obtaining a local increase in dielectrophoretic forces without having to increase the voltages applied to the biasable electrode structures.

An additional advantage of the technique of the present embodiments is the ability to provide high field gradients at many geometrical configurations of the biasable electrodes. This is because the high field gradients are local (near the floating electrodes) and it is not necessary to generate high field gradients near the biasable electrodes. Thus, unlike traditional dielectrophoresis devices for manipulating nanoparticles which are limited to polynomial geometries, the device of the present embodiments can comprise geometrical configurations other than polynomial. For example, the device of the present embodiments can manipulate nanoparticles using, but not limited to, interdigitated biasable electrode structures and/or deflection electrodes and/or castellated electrodes and/or spiral electrodes and/or sinusoidal electrodes.

An additional advantage of the technique of the present embodiments is the ability to increase the density of electrodes in the device by providing more floating electrode structures. Such configuration facilitates the simultaneous manipulation of many particles and offers a higher throughput. As the floating electrode structure is of nanometric scale and the biasable electrode structures as well as the gap between adjacent biasable electrode structures are of micrometric scale, a single gap between adjacent biasable electrode structures can be occupied with numerous floating nanoelectrode structures. Such configuration is inherently suitable for massive parallel processing.

Since the floating electrode structures are not connected to a power source, a large number and high density of electrode structures can be incorporated in the device without or with minimal additional power. Such configuration preserves relatively low Joule heating of the medium in the device. This is particularly advantageous when the manipulated objects are nanoparticles which tend to undergo Brownian motions and are much more susceptive to temperature changes relative to larger objects such as microparticles.

Reference is now made to Figure 2a which is a schematic illustration of a microfluidic device **30**, according to various exemplary embodiments of the present invention. Microfluidic device **30** typically comprises device **10** and can be used for performing many useful tasks, primarily, but not exclusively, in the field of life-science.

For example, a microfluidic device containing device **10** can be used in medical diagnostics, *e.g.*, for processing a volume of sample from a subject (such as a droplet of blood). The sample and/or other small volumes of fluids containing analytes can be moved by electrokinetics (*e.g.*, dielectrophoresis) from reservoirs or other receiving chambers through microchannels of the microfluidic device to one or more reaction or association chambers so as to determine whether the sample contains one or more target molecules of interest (such as DNA from a pathogen).

A microfluidic device containing device **10** can also be configured for use in sampling air to determine the presence of pathogens or poisons by drawing in a sample of air and processing this fluid sample to identify whether, *e.g.*, DNA or another signature of interest (such as proteins uniquely associated with the pathogen) is present.

A microfluidic device containing device **10** can also be used as a sorter or purifier, in which individual cells or molecules of interest are separated from other cells or molecules by size, type, or other criteria.

Device **10** can also be implemented in various kinds of arrays, such as, but not limited to, an oligonucleotide array, where fluids containing labeled target oligonucleotides are moved to a surface of a substrate to which complementary probe oligonucleotides are attached, or protein arrays, where fluids containing labeled proteins are moved to a surface of a substrate to which probe proteins are attached and with which the targets of interest associate.

A microfluidic device containing device **10** can also be used as a chromatograph for performing liquid chromatography.

A microfluidic device containing device **10** can also be used as a microfluidic printing device, in which inks are formed by moving precursors through microchannels.

A microfluidic device containing device **10** can also be used as a microfluidic mixer, in which one or more fluids are moved through a mixer inserted in a microchannel.

A microfluidic device containing device **10** can also be used as an optical device, in which a bubble or slug of fluid immiscible in a second fluid is moved through the second fluid to a spot of optical activity on the substrate.

Referring now again to the Figure 2a, microfluidic device **30** preferably comprises a power source device **32,** which is electrically connected to the biasable electrode structures of device **10** and configured for applying bias thereto. Power source device **32** can be an integral part of device **10** or it can be part of device **30** in which communication between devices **32** and **10** can be established by suitable connection lines, as known in the art.

Power source device **32** can be configured to provide in-phase and/or out-of-phase signals to individual members of the biasable electrodes, to generate a classical or traveling wave dielectrophoresis. Alternatively, power source device **32** can be a direct-current power source device.

In various exemplary embodiments of the invention device **30** comprises one or more inlet ports **38** and one or more outlet ports **40**. Inlet port **38** and outlet port **40** are in fluid communication with the chamber of device **10**. Optionally and preferably device **30** comprises a fluid flow driving system **42**, such as a pump or the like, for supplying fluid to inlet port **38** and removing fluid from outlet port **40**. System **42** can comprise, for example, one or more pumps, *e.g.*, micro-pumps.

In various exemplary embodiments of the invention device **30** further comprises a detector **34** for detecting the presence of the object(s) in device **10**. Many types of detectors are contemplated. In one embodiment, detector **34** detects variations in the electrical characteristics in a predetermined region **36** within the chamber of device **10**; in another embodiment, detector **34** detects variations in the optical characteristics in region **36**.

It is expected that during the life of this patent many relevant detectors will be developed and the scope of the term "detector" is intended to include all such new technologies *a priori*.

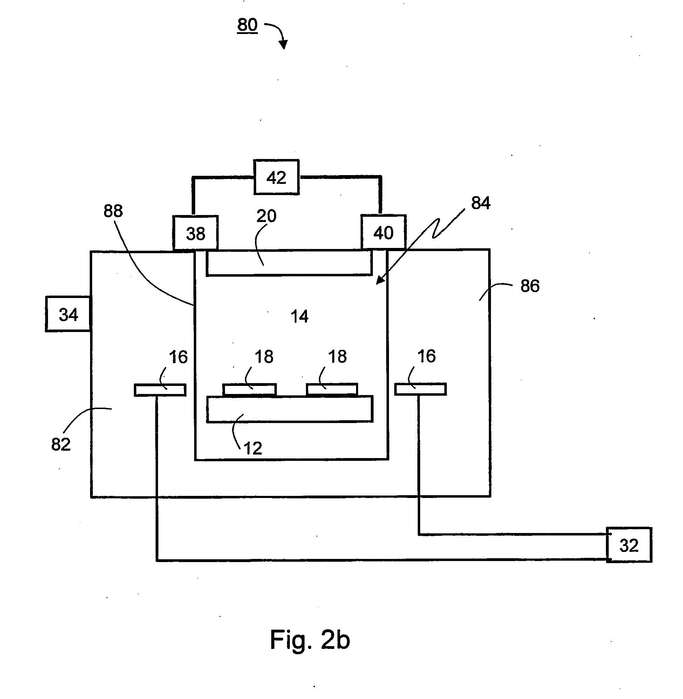

Reference is now made to Figure 2b, which is a schematic illustration of an apparatus **80** for manipulating an object present in a fluid by electrokinetics, according to various exemplary embodiments of the present invention. The principles and operations of apparatus **80** are similar to the principles and operations of device **10** and/or **30** above, except that in apparatus **80**, one or more of the electrode structures are detachable from the apparatus. This embodiment is particularly useful when it is desired to replace the electrode structures and/or the flow chamber.

Thus, according to the presently preferred embodiment of the invention apparatus **80** comprises substrate **12** having formed thereon or being integrated with one or more electrically floating electrode structures **18.** Substrate **12** forms flow chamber **14** as further detailed hereinabove. Optionally, apparatus **80** also comprises substrate **20** as further detailed hereinabove. Substrates **12** and/or **20** can be made disposable.

Apparatus **80** further comprises an electrically activable device **82**, having electrically biasable electrode structures **16** as further detailed hereinabove. Structures **16** can be formed on or integrated with a substrate **86** which is preferably made of electrically insulating or dielectric material, such as, but not limited to, glass, silicon dioxide, resistive (non-conductive) silicon, plastics (such as, but not limited to, those used for printed circuit board substrates), elastomers (*e.g.*, poly-dimethylsiloxane), insulating photoresists (*e.g.*, SU-8), ceramic or the like. For example, substrate **86** can be made of the same material as substrate **12**. Device **82** is designed and constructed to receive substrate **12** and/or **20** and to generate an electric field in a region **84** engaged by the substrate(s). Device **82** preferably serves as housing for substrate **12** and optionally substrate **20**, and typically comprises a recess or slot **88** which is sizewise and shapewize compatible with the substrate(s).

Apparatus **80** can comprise any of the aforementioned components of microfluidic device **30**, including, without limitation, power source device **32**, ports **38** and **40**, fluid flow driving system **42** and detector **34**.

The device of the present embodiments can be manufactured by fabricating a plurality of electrode structures in a chamber, which can be, for example, a substrate made of glass or any other insulating and/or dielectric material, and fabricating a plurality of electrical contacts in the chamber, such that a portion of the electrode structures are connected to the electrical contacts, and one or more electrode structures remains insulated from the contacts and the other electrodes. The electrode structures can be of similar size.

The fabrication of electrodes can be by any constructive and/or destructive fabrication technique or process known in the art, including, without limitation, evaporation, lithography, lift-off, spattering, e-beam lithography, focused ion beam milling and the like. For example, a metal, preferably Titanium followed by Gold, can be deposited on the chamber by acceleration of particles within a vacuum tube. The selection of Titanium and Gold is due to the known properties of these metals to adhere well to each other and to a glass substrate. Alternatively, an aluminum layer can be deposited on the chamber using an electron-beam evaporator.

The electrical contacts can be fabricated, for example, by patterning and evaporation of a conductive material, onto the chamber.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

EXAMPLES

Reference is now made to the following examples, which together with the above descriptions, illustrate the invention in a non limiting fashion.

***EXAMPLE 1***

***Mathematical Formulae***

A particle subjected to a nonuniform electric field ($\underline{E}$) experiences polarization. The electric force ($F_{elect}$) acting upon the particle is a function of the field distribution and the dielectric polarization components induced in the particle by the field. If the particle is neutral or an alternating field whose time average is zero is applied, the electric force resulting from net charge vanishes. In this case, the dipolar moment induced in the particle and the field gradient values dominate the electric force. The resulting force can be approximated as:

$$\langle \underline{F}_{elect} \rangle = 2\pi\varepsilon_m R^3 \left[ \mathrm{Re}\{f_{CM}\} \nabla \underline{E}_{rms}^2 + \mathrm{Im}\{f_{CM}\} \left( E_x^2 \nabla\varphi_x + E_y^2 \nabla\varphi_y + E_z^2 \nabla\varphi_z \right) \right],$$

$$(\text{EQ. 1})$$

where $\langle x \rangle$ is the time averaged value of $x$, $\varepsilon_m$ is the medium permittivity, $R$ is the particle radius, Re$\{x\}$ and Im$\{x\}$ are the real and imaginary components of $x$ respectively, $\nabla$ is the gradient operator, $\underline{E}_{rms}$ is the root mean square electric field,

$E_i$ is the electric field component in the direction i, $\varphi_i$, is the phase of the electric field component $E_i$ and $f_{CM}$ is the frequency ($\omega=2\pi f$) dependent Clausius-Mossotti factor of the first order:

$$f_{CM}(\omega) = \frac{\varepsilon_p^* - \varepsilon_m^*}{\varepsilon_p^* + 2\varepsilon_m^*}, \qquad \text{(EQ. 2)}$$

$\varepsilon_p^*, \varepsilon_m^*$ are the complex conjugate permittivities of the particle and the medium, respectively:

$$\varepsilon_p^* = \varepsilon_p - j\frac{\sigma_p}{\omega} \;\; ; \;\; \varepsilon_m^* = \varepsilon_m - j\frac{\sigma_m}{\omega}, \qquad \text{(EQ. 3)}$$

$j^2 = -1$, $\sigma_p, \sigma_m$ are the conductivities of the particle and the medium respectively and $\varepsilon_p$ is the particle permittivity.

The dielectrophoretic force $F_{DEP}$ exists when the intensity and/or the phase of the applied electric field is nonuniform. The classic dielectrophoretic force is proportional to the intensity gradient $\nabla E_{rms}^2$ (first term of Equation 1) and the traveling-wave dielectrophoretic force is proportional to the phase gradient $\nabla\varphi_i$ (second term in Equation 1).

Generally, the dielectrophoretic force depends on the particle volume ($R^3$). The direction of the dielectrophoretic force depends on the polarity of the induced dipolar moment which is determined by the conductivities and permittivities of the particle and its suspending medium, as given by Equation 2 above. The dielectrophoretic force is highly selective. It can change significantly for particles that are not very different from each other, such as viable and nonviable cells.

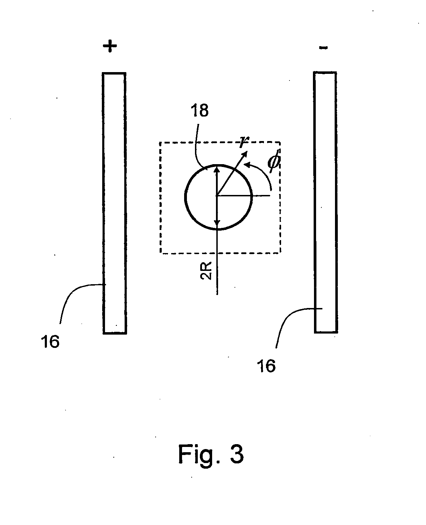

Reference is now made to Figure 3 which is a schematic illustration of device **10** in an exemplified embodiment in which the device comprises two electrically biasable electrode structures **16** and a single cylindrical floating electrode structure **18**. In the absence of structure **18**, the electric field between structures **16** is uniform: $E = E_0$.

The cylindrical floating electrode structure alters the electric field in the device. The expressions for the electric field and the field intensity gradient become:

$$E = E_0\left[1+\left(\frac{R}{r}\right)^2\right]\cos\phi\hat{i}_r - E_0\left[1-\left(\frac{R}{r}\right)^2\right]\sin\phi\hat{i}_\phi \qquad \text{(EQ. 4)}$$

$$\nabla E^2 = -4E_0^2\left[\cos 2\phi\frac{R^2}{r^3}+\frac{R^4}{r^5}\right]\hat{i}_r - 4E_0^2\frac{R^2}{r^3}\sin 2\phi\hat{i}_\phi, \qquad \text{(EQ. 5)}$$

where $R$ is the floating cylinder radius, $r$ is the radial coordinate, $\varphi$ is the angular coordinate, $\hat{i}_r$, is the radial unit vector and $\hat{i}_\phi$, is the angular unit vector (see Figure 3).

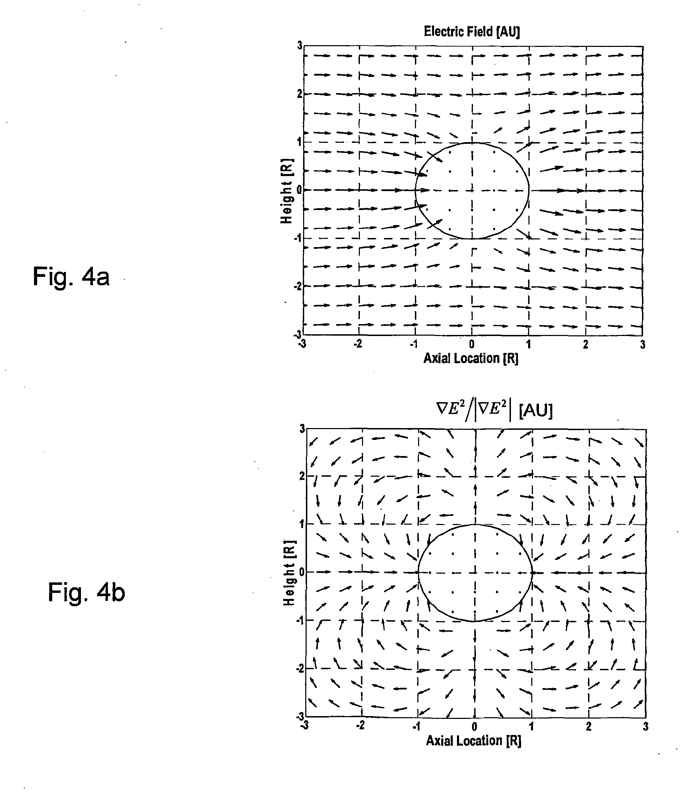

Figures 4a-b illustrate the electric field and the normalized field intensity gradients $\nabla E^2 / \left|\nabla E^2\right|$ as obtained from Equations 4 and 5, respectively. The results are displayed in the vicinity of the cylindrical floating electrode (the dashed rectangle shown in Figure 3). The geometric dimensions of the model are normalized by the radius of the cylinder $R$.

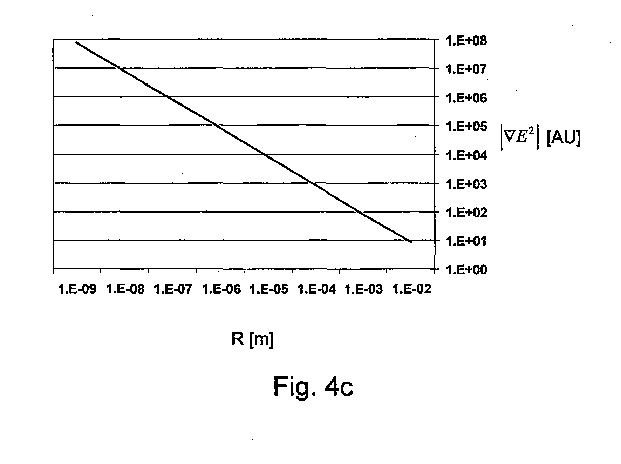

Figure 4c shows the field intensity gradients in the vicinity of the cylindrical floating electrode structure as a function of the radius of the cylinder $R$. As shown, the field intensity gradient is inversely proportional to $R$. Thus, dielectrophoretic forces in the vicinity of a nanoscale floating cylinder are expected to be three orders of magnitude larger than those in the vicinity of a microscale cylinder.

*EXAMPLE 2*

*Simulations*

Computer simulations of the electrical field within two types of model devices were performed.

## Methods

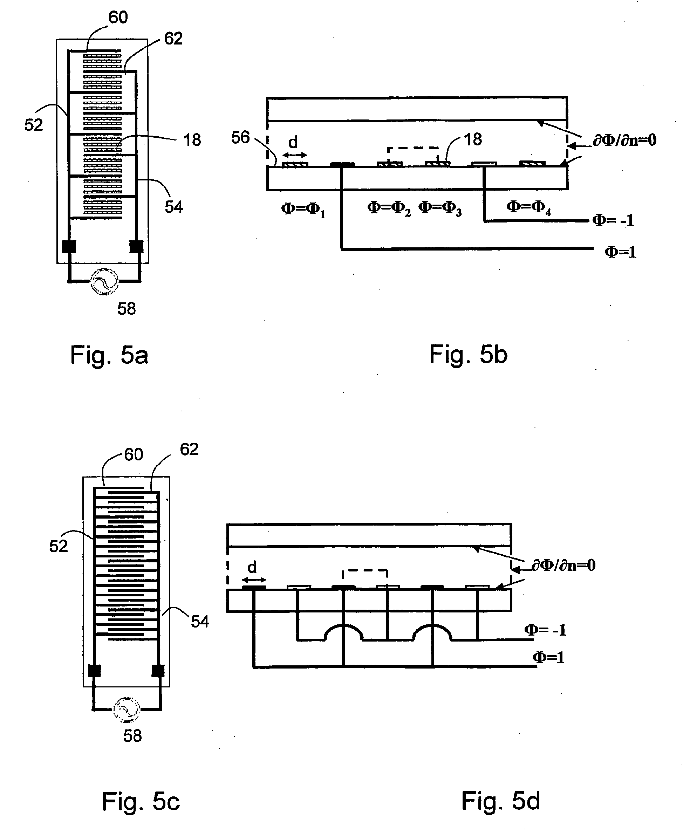

The model devices are schematically illustrated in Figures 5a-d. A first mdel device, illustrated in Figures 5a (top view) and 5b (fragmentary side view) was designed according to a preferred embodiment of the present invention and included both electrically biasable electrode structures and electrically floating electrode structures. The electrically biasable electrode structures were arranged in an interdigitated arrangement

As illustrated in Figure 5a, the electrically biasable electrode structures comprise first electrode stem **52**, disposed proximate to the substrate surface **56** and parallel with a second electrode stem **54**, proximate to the same surface. The first electrode stem is connected to a first terminal of an AC power source **58**, and the second electrode stem is connected to a second terminal of power source **58**, such that that the first electrode stem and the second electrode stem have opposite polarities. A series of first electrode "digits" **60** extend in a substantially normal direction from first electrode stem **52** towards second electrode stem **54**, without touching the second electrode stem. Similarly, a series of "digits" **62** of second electrode stem **54** extend in a substantially normal direction from the second electrode stem towards the first electrode stem, without touching the first electrode stem. First electrodes digits **60** are spaced so that the digits are adjacent to and substantially parallel with second electrode digits **62**. As a result of the alternating arrangement of interdigitated electrodes **60** and **62**, an electrode having one polarity at a given moment is adjacent to one or more electrodes having the opposite polarity.

The electrically floating electrodes **18** are disposed in the gaps between adjacent electrode digits **60**, **62**. Simulations were performed both for floating electrodes characterized by micrometric dimensions and for floating electrodes characterized by nanometric dimensions. The configuration illustrated in Figures 5a-b is referred to hereinunder as the floating electrode dielectrophoresis (feDEP) configuration.

A second model device, illustrated in Figures 5c (top view) and 5d (fragmentary side view) included only electrically biasable electrode structures arranged in an interdigitated arrangement. The configuration illustrated in Figures 5c-d is referred to hereinunder as the traditional dielectrophoresis configuration.

In both model devices, a phase sequence of half a cycle was applied to the biasable electrodes. The biasable electrodes were simulated as being applied by alternating voltages of amplitude $V_0$. The voltages were used as boundary conditions for the simulations. The voltages were normalized so as to provide a dimensionless potential $\Phi$, defined as the applied voltage divided by $V_0$. Thus, $\Phi$ alternates between 1 and -1 at the electrodes.

The model devices for floating electrodes characterized by micrometric dimensions included nine electrodes, enumerated serially from 1 to 9. In the traditional dielectrophoresis configuration, all nine electrodes were biased, and in the floating electrode dielectrophoresis configuration electrode Nos. 3 and 7 were biased and electrodes Nos. 1, 2, 4, 5, 6, 8 and 9 were floating electrodes. The model devices for floating electrodes characterized by nanometric dimensions included eight electrodes, enumerated serially from 1 to 8. Electrode Nos. 3 to 6 were floating and electrodes Nos. 1, 2, 7 and 8 were biased electrodes.

An electric isolation condition was imposed as a Dirichlet boundary condition of zero normal electric field ($\partial\Phi/\partial n = 0$) at all boundaries other than the electrodes. The floating electrodes in the first device were modeled as equipotential perfect electric conductors, and zero tangential electric field was imposed thereat. Additionally, a zero net charge was imposed on each floating electrode. The dimensionless potentials at different floating electrodes (denoted in Figure 5b by ($\Phi_i$, i = 1, 2,...) were initially unknown and were calculated by finite element method. The equipotential constraints at the floating electrode were therefore not Dirichlet boundary conditions.

A commercial finite element simulation software ANSYS® was used for calculating the electric fields. MATLAB® software was used for studying and visualizing the field intensity gradients.

The electric field generated in the configuration of Figures 5c-d is spatially periodic and can be solved by modeling only a single electrode. Yet, to avoid numeric discrepancies that may occur when a finite element mesh is changed, a single mesh was maintained for all the microscale floating electrode configurations studied. Unlike the configuration in Figure 5c-d, there is a-priori no spatial periodicity when floating electrodes are incorporated. The mesh therefore contained all the nine electrodes used. The geometric dimensions of the models were normalized by the characteristic length of the electrode width, indicated by *d* in Figures 5b and 5d.

## Results

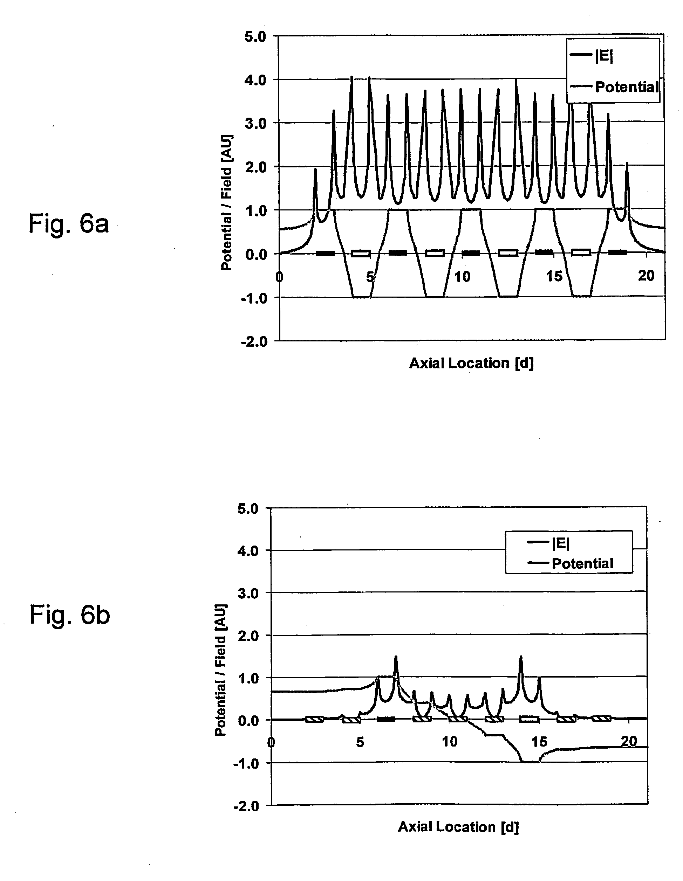

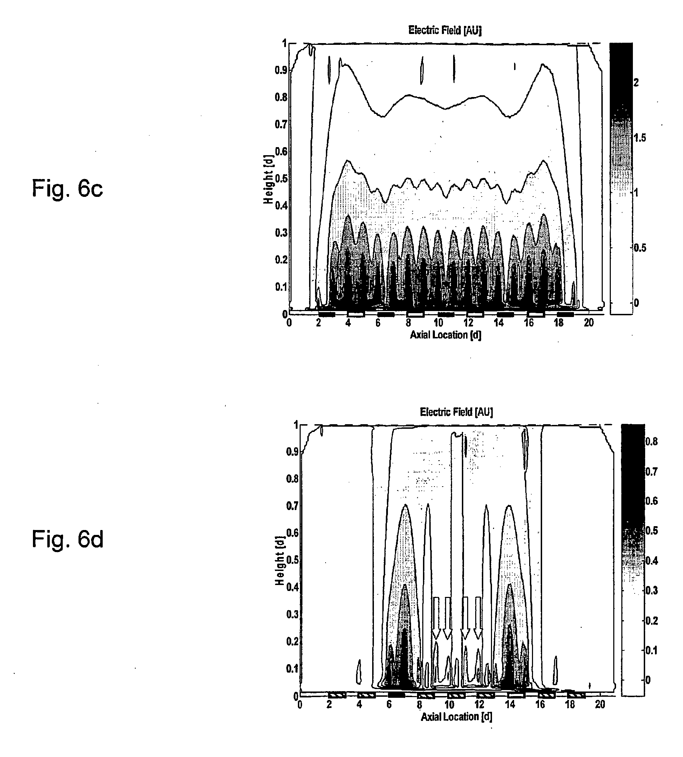

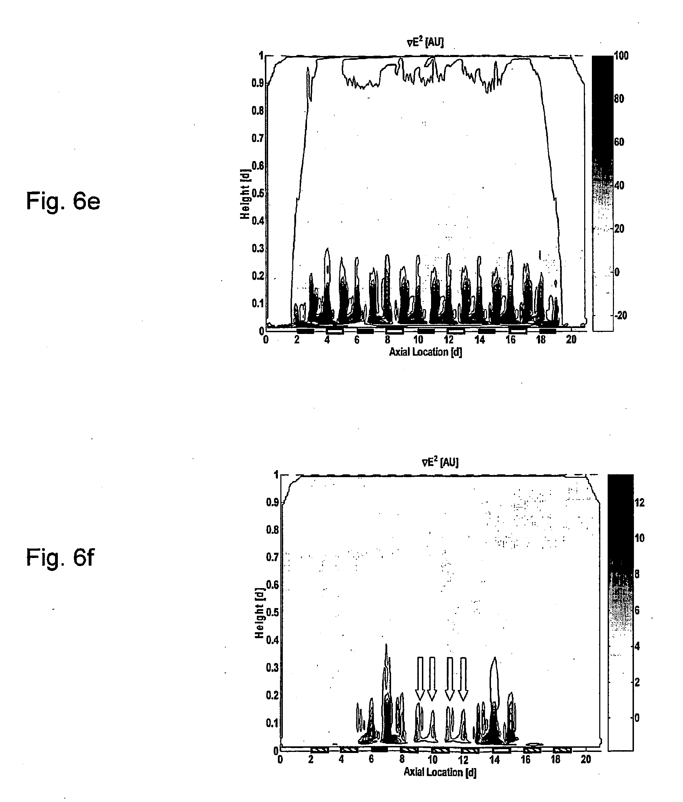

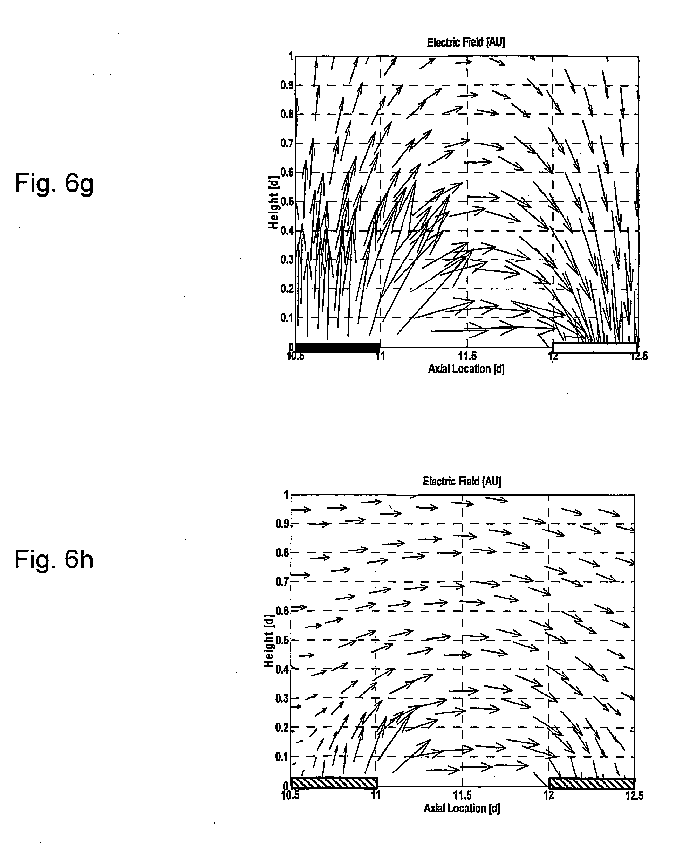

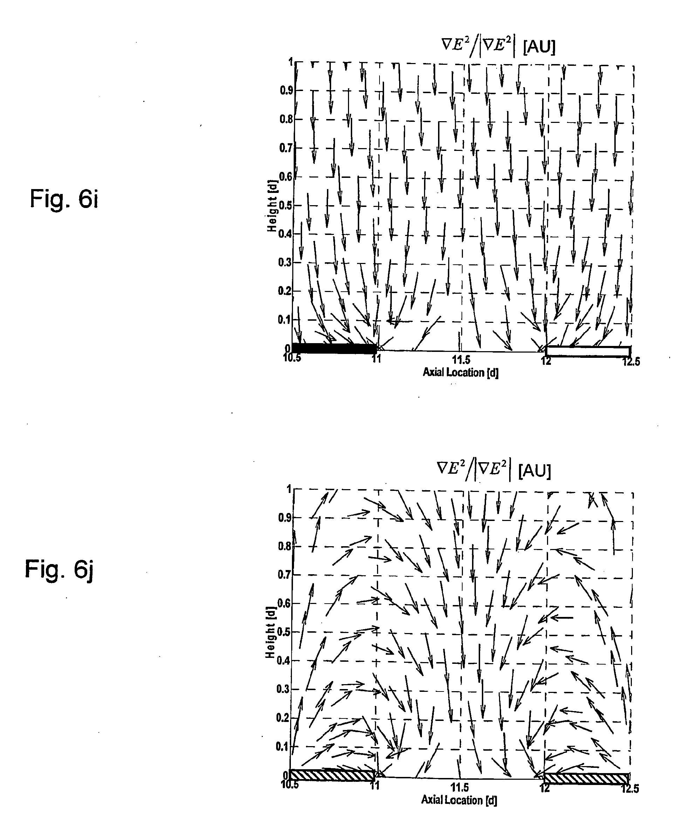

The numerical calculations for the microscale floating electrode case are presented in Figures 6a-j, where Figures 6a-b show the dimensionless potential and the electric field intensity (|E|) along the electrode plane for the traditional (Figure 6a) and floating electrode (Figure 6b) configuration; Figures c-d show iso-contours of the field intensity for the traditional (Figure 6c) and floating electrode (Figure 6d) configuration; Figures 6e-f show iso-contours of the field intensity gradient for the traditional (Figure 6e) and floating electrode (Figure 6f) configuration; Figures 6g-h show vector representation of the electric field between two adjacent electrode centers for the traditional (Figure 6g) and floating electrode (Figure 6h) configuration; and Figures 6i-j show vector representation of the normalized field intensity gradient between two adjacent electrode centers for the traditional (Figure 6i) and floating electrode (Figure 6j) configuration.

Referring to Figures 6a, 6c, 6e, 6g and 6i (traditional dielectrophoresis configuration), the potential alternates between 1 and -1 and the changes are nearly linear between the electrodes (Figure 6a). The electric field increases as it approaches the electrode plane (Figure 6c). It is normal to the plane at the electrode and tangential to the plane at the glass substrate (Figure 6g). The highest field intensities and field intensity gradients appear at electrode edges (Figures 6c and 6e). It is noted that Equation 1 shows that the field intensity gradient represents the orientation of the dielectrophoretic force. The force is therefore normal to the electrode plane, directed downward and points towards electrode edges near the metallization (Figure 6i).

Figures 6b, 6d, 6f, 6h and 6j show the simulation results for the floating electrode dielectrophoresis configuration. The field intensities and field intensity gradients are substantially different from those of the traditional dielectrophoresis configuration.

In the floating electrode dielectrophoresis configuration, only the third and seventh electrodes from the left are biased with external voltage. The remaining electrodes are floating electrodes. The potential reaches values of 1 and -1 at the biased electrodes 3 and 7, respectively (Figure 6b). The floating electrodes are equipotential. Floating electrode Nos. 4-6 which are located between the biased electrodes are indicated by arrows. The potential values of floating electrode Nos. 4-6 exhibit a nearly linear decrease from 1 to -1.

The potential values of the floating electrode Nos. 1-2 and 8-9 which are located outside the biased electrodes are also affected. For these electrodes, however, the potential values do not exhibit linear behavior. The electric field increases as it approaches the electrode plane (Figure 6d).

The highest field intensity values were obtained at the biased electrode edges (Figure 6f). Nevertheless, the floating electrodes were also affected and exhibited high field intensity values at their edges, as indicated by the arrows. The edges of floating electrode Nos. 4-6 are indicated by arrows. These floating electrodes were more affected than the floating electrodes outside the biased electrodes.

The field between two adjacent floating electrodes was normal to the plane at the electrode and tangential to the plane at the glass substrate (Figure 6h). However, the dielectrophoretic force distribution at the floating electrodes was different (Figure 6j). The force was directed upwards above the electrodes and downwards therebetween. In the vicinity of the electrode, the force points towards the electrode edges. Such a force distribution tends to move the particles positioned above electrodes up and move the particles positioned between electrodes down. Yet, all the particles are expected to collect at the electrode edges.

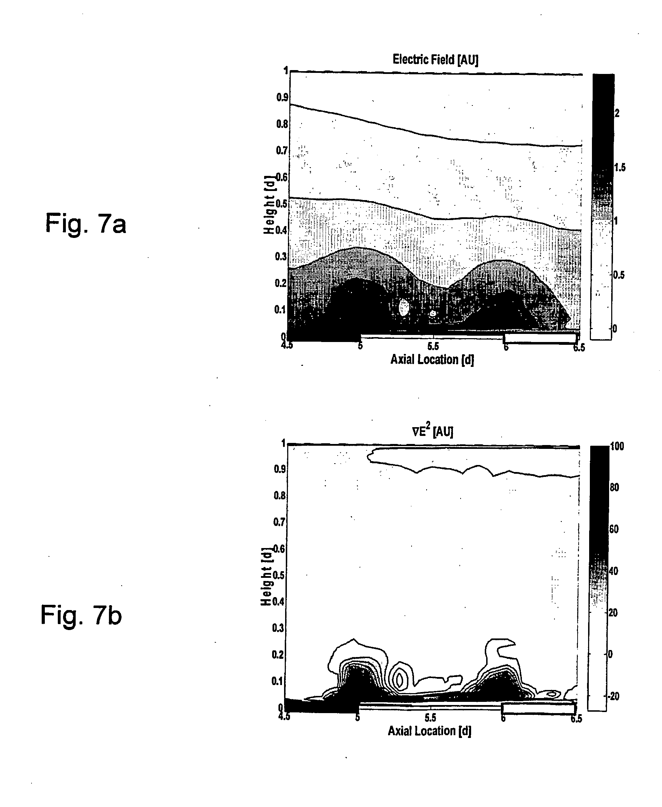

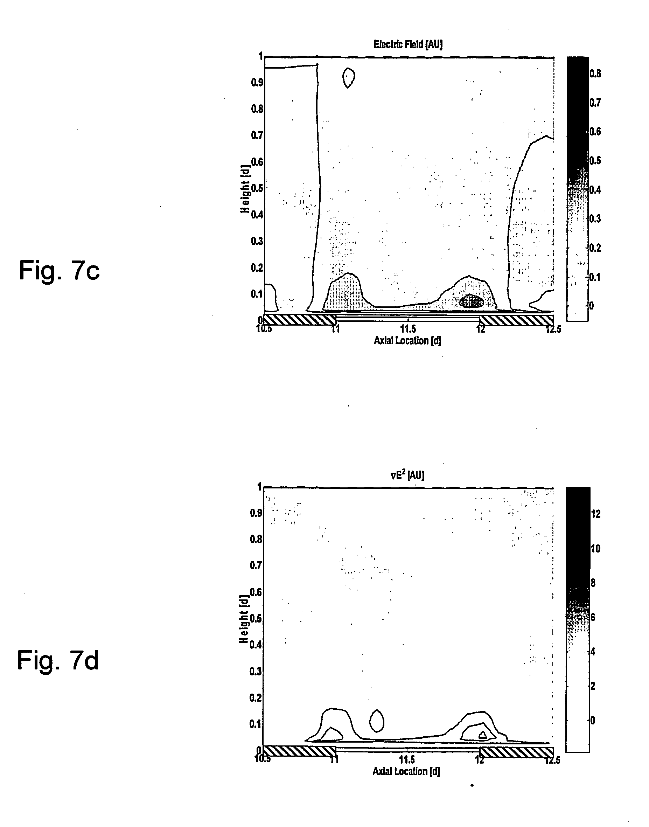

Figures 7a-b show the results of finite element calculations for the traditional dielectrophoresis device. The results relate to a representative region between two adjacent biased microelectrode centers (the dashed rectangle illustrated in Figure 5d). The filled and empty bars at the bottom of each figure represent the biased electrodes (as illustrated in Figure 5d). The highest field intensities and field intensity gradients were obtained at the edges of the biased electrodes.

Figures 7c-d show the results of finite element calculations for the floating electrode dielectrophoresis configuration in which the floating electrodes were simulated as having micrometric size not in accordance with the present invention. The results relate to a representative region between two adjacent floating electrode centers (the dashed rectangle seen in Figure 5b). The patterned bars at the bottom of each figure represent the floating electrodes (as illustrated in Figure 5b). The floating electrode dielectrophoresis configuration resulted in field intensities and field intensity gradients that are different from those of the traditional dielectrophoresis configuration. Nevertheless, the floating electrodes also exhibited the highest field intensities and field intensity gradients at their edges. Therefore, particles are expected to collect at the floating electrode edges as well as at biased electrode edges.

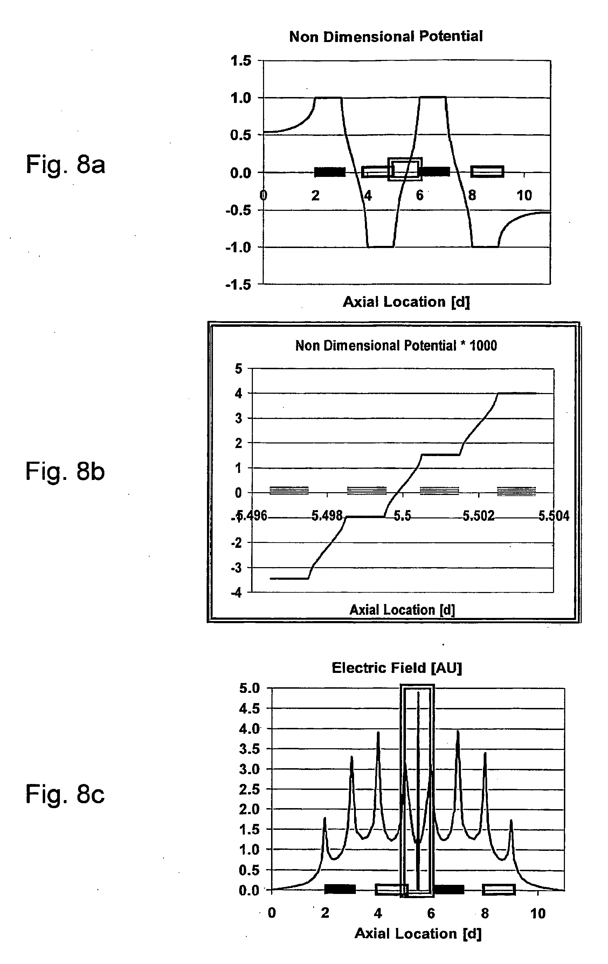

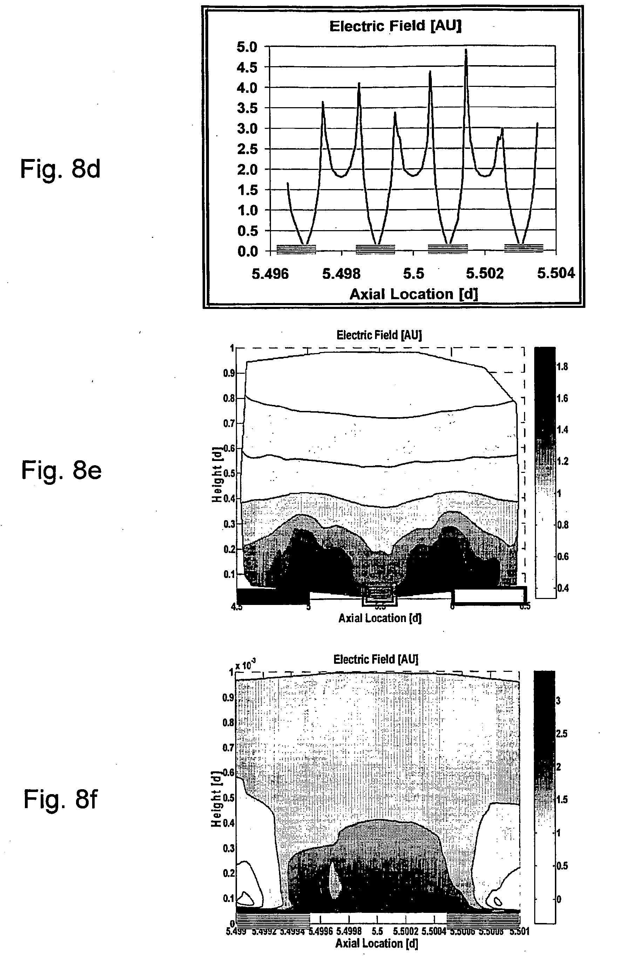

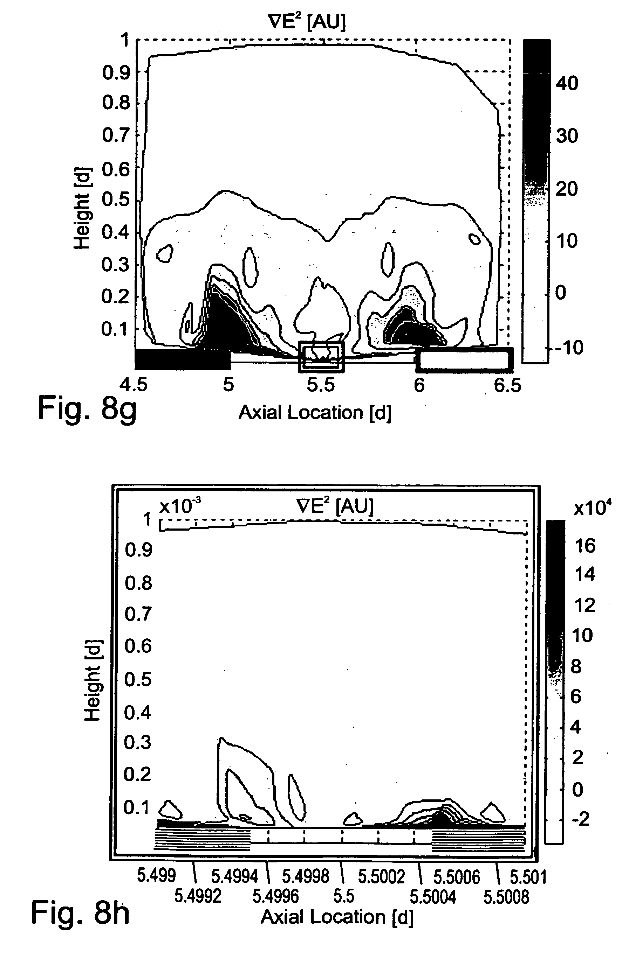

Figures 8a-h show the results of finite element calculations for the floating electrode dielectrophoresis configuration in which the floating electrode were simulated as having nanometric size. The results are displayed on two different size scales. The scale in Figures 8a, 8c, 8e and 8g represents the microscale spacing between the biased electrodes, and the scale in Figures 8b, 8d, 8f and 8h represents the nanoscale spacing between the floating nanoelectrodes.

As shown in Figure 8a, the dimensionless potential alternated between 1 and -1 at the biased microelectrodes. As shown in Figure 8b, the floating nanoelectrodes were equipotential, and the potential at each floating nanoelectrode was unique and different from the potential at other floating nanoelectrodes. Thus, $\Phi_i \neq \Phi_j$ for $i \neq j$. The potential values of the nanoelectrodes were determined by capacitive coupling to the biased microelectrodes, and are consistent with the spatial distribution of the potential imposed by the biased microelectrodes. For the region presented in Figure 8b, located approximately in the middle between adjacent biased microelectrodes, the potential is expected to be nearly zero.

Figures 8c-d show the obtained electric field values. Maximal values of the electric field were obtained at the electrode edges. There was no significant difference between field intensity values at the biased microelectrodes and field intensity values at the floating nanoelectrodes.

Figures 8e-h show the electric field intensity and field intensity gradient distributions. In Figures 8e and 8g, the electric field intensity and field intensity gradient distributions are shown for a region between adjacent biased microelectrode centers (microscale region). In Figures 8f and 8h the electric field intensity and field intensity gradient distributions are shown for a region between adjacent floating nanoelectrode centers (nanoscale region). In both the microscale and nanoscale regions, the highest field intensities and field intensity gradients were obtained at the electrode edges. The electric field values at the biased microelectrodes and at the floating nanoelectrodes are similar (Figures 8e-f). Still, the field intensity gradients at the floating nanoelectrodes were increased by a factor of 2500 in comparison to those at the biased microelectrodes (Figures 8g-h).

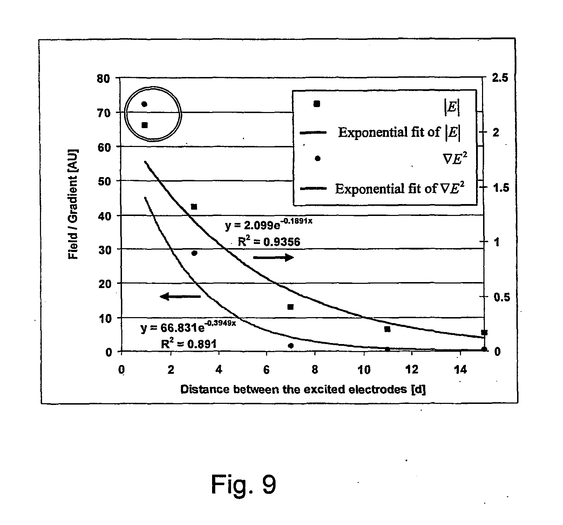

Figure 9 shows the effect of the distance between the biased electrodes on the field intensity and field intensity gradient at the floating electrode edges, for the floating electrode dielectrophoresis configuration in which the floating electrodes were simulated as having micrometric size. The distance between the biased electrodes is a function of the number of floating electrodes located between the biased electrodes. In Figure 9, the obtained values for the field intensity are shown as filled squares, and the obtained values for field intensity gradient are shown as filled circles. Also shown are curves representing numerical fits to the obtained values. The field intensity and field intensity gradient decreased generally exponentially with the distance between the biased electrodes. For the field intensity, the numerical fit was $2.099 \exp(-0.1891x)$, with a squared Pearson coefficient of 0.9356, and for the field intensity gradient, the numerical fit was $66.831 \exp(-0.3949x)$, with a squared Pearson coefficient of 0.891, where x is the distance between the biased electrodes normalized to the electrode width d as defined in Figures 5b and 5d.

## *EXAMPLE 3*

### *Prototype Device*

A prototype device was manufactured in accordance with preferred embodiments of the present invention. The prototype device was used for manipulating erythrocytes in blood sample.

### **Materials and Methods**

Two different electrode configurations were fabricated. The two electrode configurations had different feature sizes and were fabricated using different processes and materials.

In a first configuration, each electrode was 12.5 $\mu$m in width, and adjacent electrodes were separated by a 12.5 $\mu$m gap. A 200-Å-thick titanium and a 2000-Å-thick gold layers were subsequently deposited on a microscope slide using an electron-beam evaporator. The Ti/Au electrodes were obtained using a lift-off process. The obtained device included various electrode layouts in an arrangement in which 7-8 floating electrodes were located between or adjacent to two biasable electrodes. The number of the floating electrodes and their arrangement in relation to the biased electrodes was controlled by changing the bias connection from electrode to electrode within the array.

In a second configuration, each electrode was 25 $\mu$m in width and adjacent electrodes were separated by a 25 $\mu$m gap. A 7000-Å-thick aluminum layer was deposited on a soda lime glass wafer using an electron-beam evaporator. The electrodes were obtained using an aluminum etching process. The obtained device included an electrode arrangement in which 7-8 floating electrodes were located between or adjacent to two biasable electrodes. The number of the floating electrodes and their arrangement in relation to the biased electrodes was controlled by changing the bias connection from electrode to electrode within the array.

Fresh whole blood was obtained from white male Sprague Dawley rats. All animals were older than three months and weighed about 250 grams. The blood was drawn from the aorta prior to animal sacrifice into a syringe washed with an anticoagulant (héparine choay, 5000 U.I/1 ml). A suspending buffer was prepared by diluting PBS (Dulbecco's Phosphate Buffered Saline - D8662, Sigma Aldrich) with deionized water to give a range of conductivities. Standard dilution of the blood was performed by using the suspending buffer at a 1:100 ratio. The conductivity of the sample was measured using a conductivity meter (Fluke 179).

The devices were contacted by the sample and a 1 MHz, 10 V (peak to peak) voltage was applied to the biasable electrodes.

Erythrocyte motion was visually recorded with a Nikon VM Lens adapter on a Nikon SMZ800 microscope equipped with a Sony SSC-M370CE high resolution CCD camera. Digital output from the camera was routed to a computer with a National Instruments IMAQ PCI 1411 video capture card. LabVIEW 7.1 software (National Instruments) was used for controlling the recording parameters. AC voltage was applied to the electrodes from an Agilent HP33220A function generator. Waveforms were monitored with a Tektronix TDS 1002-60 MHz oscilloscope.

**Results**

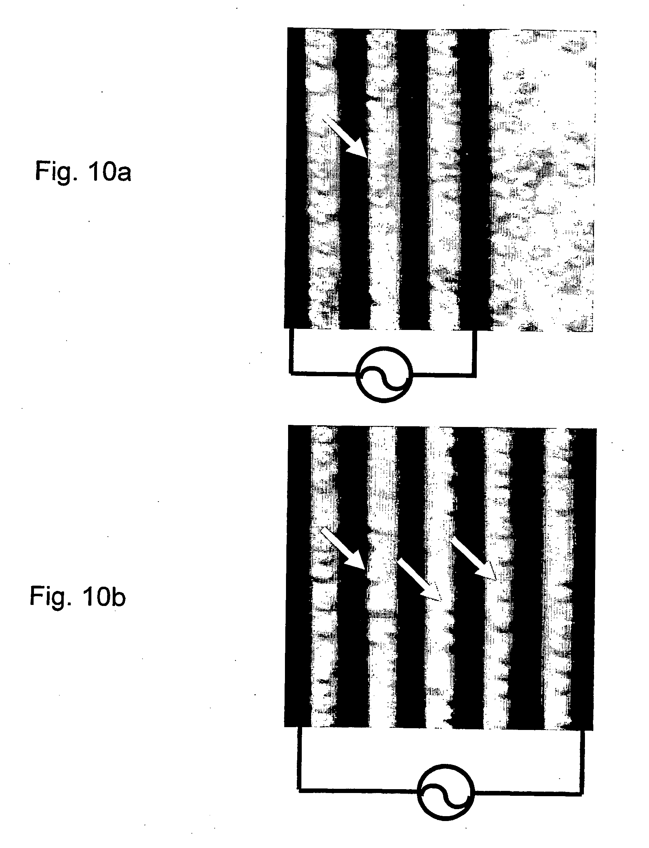

Figures 10a-b show two video images captured in the experiments. The biased electrodes are marked by solid lines symbolizing the function generator and the voltage connections. The remaining electrodes are floating electrodes. The images in the figures correspond to the first prototype device (Ti/Au electrodes, lift-off technique). Figures 10a-b demonstrate erythrocyte collection at floating electrode edges, as indicated by white arrows, thus demonstrating that the erythrocytes experienced positive dielectrophoresis.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

**Claims**

1. Apparatus (80) for manipulating an object present in a fluid by electrokinetics, the apparatus (80) comprising:

   a substrate (12) forming a flow chamber (14) and having formed thereon or being integrated with at least one electrically floating electrode structure (18) having at least one nanometric dimension; and
   an electrically activable device (82), having a plurality of electrically biasable electrode structures (16) and being designed and constructed to receive said substrate (12) and to generate an electric field in a region engaged by said substrate (12), such that in operation there is no bias which is applied to said at least one electrically floating electrode structure (18).

2. The apparatus (80) of claim 1, wherein at least one of said electrically biasable electrode structures (16) is **characterized by** at least one micrometric dimension.

3. The apparatus (80) of any of claim 1 and 2, wherein said at least one electrically floating electrode structure (18) is designed and constructed to increase a dielectrophoretic force exerted on the object by at least an order of magnitude.

4. The apparatus (80) of any of claim 1 and 2, wherein said at least one electrically floating electrode structure (18) is designed and constructed to increase a dielectrophoretic force exerted on the object by at least N orders of magnitude, said N being at least 2.

5. The apparatus (80) of any of claims 1-4, wherein the device further comprises at least one additional electrically floating electrode structure (18),

6. The apparatus (80) of any of claim 1-5, wherein said plurality of electrically biasable electrode structures. (16) comprises interdigitated electrodes.

7. The apparatus (80) of any of claims 1-6, wherein said electrically floating electrode structures (18) comprise carbon nanotubes.

8. The apparatus (80) of any of claims 1-7, further comprising a power source device (32), electrically connected to said plurality of electrically biasable electrode structures (16) and configured for applying bias thereto.

9. The apparatus (80) of claim 8, wherein said power source device (32) is configured to provide out-of-phase signals to individual members of said plurality of electrodes.

10. The apparatus (80) of claim 9, wherein said out-of-phase signals are selected such as to generate a traveling wave dielectrophoretic force.

11. The apparatus (80) of claim 9, wherein said out-of-phase signals arc selected such as to generate a classical dielectrophoretic force,

12. A method of manipulating an object present in a fluid by electrokinetics, comprising contacting the fluid with the apparatus (80) according to any of claims 1-11, and applying bias to said plurality of electrically biasable electrode structures (16) so as to generate a nonuniform electric field, thereby manipulating the object.

13. The method according to claim 12, wherein said fluid is selected from the group consisting of water, a bacterial cell suspension, a protein medium, an antibody medium, a nucleic acid medium, ink, phosphate buffered saline, and a biological fluid.

14. The method according to claim 12, wherein said object is selected from the group consisting of a cell, a cell aggregate, a normal erythrocyte, an abnormal erythrocyte, a fetal nucleated red blood cell, a cancer cell, a protein, DNA, RNA, an antibody, an antigen, a lipid, an assemblage of molecules, a virus, a plasmid, a bacterium, a protozoan, a mineral, a crystal, a conductive particle, a semiconductive particle, a dielectric particle, and a gas bubble.

**Patentansprüche**

1. Vorrichtung (80) zum Manipulieren eines in einem Fluid vorhandenen Gegenstands durch Elektrokinetik, wobei die Vorrichtung (80) Folgendes umfasst:

   ein Substrat (12), das eine Strömungskammer (14) bildet und auf dem mindestens eine elektrisch potentialfreie Elektrodenstruktur (18) mit mindestens einer nanometrischen Abmessung gebildet oder damit integriert ist; und eine elektrisch aktivierbare Vorrichtung (82) mit mehreren elektrisch vorspannbaren Elektrodenstrukturen (16), die dazu gestaltet und ausgeführt ist, das genannte Substrat (12) aufzunehmen und ein elektrisches Feld in einer Region zu erzeugen, mit der das genannte Substrat (12) in Eingriff steht, so dass in Betrieb keine Vorspannung vorliegt, die an die genannte mindestens eine elektrisch potentialfreie Elektrodenstruktur (18) angelegt ist.

2. Vorrichtung (80) nach Anspruch 1, wobei mindestens eine der genannten elektrisch vorspannbaren Elektrodenstrukturen (16) durch mindestens eine mikrometrische Abmessung gekennzeichnet ist.

3. Vorrichtung (80) nach einem der Ansprüche 1 und 2, wobei die genannte mindestens eine elektrisch potentialfreie Elektrodenstruktur (18) dazu gestaltet und ausgeführt ist, eine auf den Gegenstand ausgeübte dielektrophoretische Kraft um mindestens eine Größenordnung zu erhöhen.

4. Vorrichtung (80) nach einem der Ansprüche 1 und 2, wobei die genannte mindestens eine elektrisch potentialfreie Elektrodenstruktur (18) dazu gestaltet und ausgeführt ist, eine auf den Gegenstand ausgeübte dielektrophoretische Kraft um mindestens N Größenordnungen zu erhöhen, wobei das genannte N mindestens 2 ist.

5. Vorrichtung (80) nach einem der Ansprüche 1-4, wobei die Vorrichtung weiter mindestens eine zusätzliche elektrisch potentialfreie Elektrodenstruktur (18) umfasst.

6. Vorrichtung (80) nach einem der Ansprüche 1-5, wobei die genannten mehreren elektrisch vorspannbaren Elektrodenstrukturen (16) interdigitierte Elektroden umfassen.

7. Vorrichtung (80) nach einem der Ansprüche 1-6, wobei die genannten elektrisch potentialfreien Elektrodenstrukturen (18) Kohlenstoffnanoröhren umfassen.

8. Vorrichtung (80) nach einem der Ansprüche 1-7, weiter umfassend eine Leistungsquellenvorrichtung (32), die elektrisch mit den genannten mehreren elektrisch vorspannbaren Elektrodenstrukturen (16) verbunden ist und dazu ausgebildet ist, Vorspannung an diese anzulegen.

9. Vorrichtung (80) nach Anspruch 8, wobei die genannte Leistungsquellenvorrichtung (32) dazu ausgebildet ist, phasenverschobene Signale an Einzelne der genannten mehreren Elektroden bereitzustellen.

10. Vorrichtung (80) nach Anspruch 9, wobei die genannten phasenverschobenen Signale derart ausgewählt sind, dass sie eine dielektrophoretische Wanderwellenkraft erzeugen.

11. Vorrichtung (80) nach Anspruch 9, wobei die genannten phasenverschobenen Signale derart ausgewählt sind, dass sie eine klassische dielektrophoretische Kraft erzeugen.

**12.** Verfahren zum Manipulieren eines in einem Fluid vorhandenen Gegenstands durch Elektrokinetik, umfassend das Berühren des Fluids mit der Vorrichtung (80) nach einem der Ansprüche 1-11 und das Anlegen von Vorspannung an die genannten mehreren elektrisch vorspannbaren Elektrodenstrukturen (16), um ein uneinheitliches elektrisches Feld zu erzeugen, wodurch der Gegenstand manipuliert wird.

**13.** Verfahren nach Anspruch 12, wobei das genannte Fluid aus der Gruppe ausgewählt wird, die besteht aus: Wasser, einer Bakterienzellensuspension, einem Proteinmedium, einem Antikörpermedium, einem Nukleinsäuremedium, Tinte, phosphatgepufferter Salzlösung und einem biologischen Fluid.

**14.** Verfahren nach Anspruch 12, wobei der genannte Gegenstand ausgewählt ist aus der Gruppe, die besteht aus: einer Zelle, einer Zellenanhäufung, einem normalen Erythrozyt, einem abnormalen Erythrozyt, einer fötalen kernhaltigen roten Blutzelle, einer Krebszelle, einem Protein, DNA, RNA, einem Antikörper, einem Antigen, einem Lipid, einer Ansammlung von Molekülen, einem Virus, einem Plasmid, einem Bakterium, einem Protozoon, einem Mineral, einem Kristall, einem leitfähigen Partikel, einem halbleitenden Partikel, einem dielektrischen Partikel und einer Gasblase.

**Revendications**

**1.** Appareil (80) pour manipuler un objet présent dans un fluide par électrocinétique, l'appareil (80) comprenant :

un substrat (12) formant une chambre d'écoulement (14) et sur lequel est formée ou intégrée au moins une structure d'électrode (18) flottant électriquement, ayant au moins une dimension nanométrique ; et
un dispositif électriquement activable (82) ayant une pluralité de structures d'électrode (16) électriquement polarisables et élaboré et construit pour recevoir ledit substrat (12) et pour générer un champ électrique dans une région où s'engage ledit substrat (12) de sorte que, en service, il n'y ait pas de polarisation qui soit appliquée à ladite au moins une structure d'électrode (18) flottant électriquement.

**2.** Appareil (80) selon la revendication 1, dans lequel au moins l'une desdites structures d'électrode (16) électriquement polarisables est **caractérisée par** au moins une dimension micrométrique.

**3.** Appareil (80) selon l'une quelconque des revendications 1 et 2, dans lequel ladite au moins une structure d'électrode (18) flottant électriquement est conçue et construite pour augmenter une force diélectrophorétique exercée sur l'objet d'au moins un ordre de grandeur.

**4.** Appareil (80) selon l'une quelconque des revendications 1 et 2, dans lequel ladite au moins une structure d'électrode (18) flottant électriquement est conçue et construite pour augmenter une force diélectrophorétique sur l'objet d'au moins N ordres de grandeur, ledit N étant d'au moins 2.

**5.** Appareil (80) selon l'une quelconque des revendications 1 à 4, dans lequel le dispositif comprend en outre au moins une structure d'électrode (18) flottant électriquement supplémentaire.

**6.** Appareil (80) selon l'une quelconque des revendications 1 à 5, dans lequel ladite pluralité de structures d'électrode électriquement polarisables (16) comprend des électrodes interdigitées.

**7.** Appareil (80) selon l'une quelconque des revendications 1 à 6, dans lequel lesdites structures d'électrode (18) flottant électriquement comprennent des nanotubes de carbone.

**8.** Appareil (80) selon l'une quelconque des revendications 1 à 7, comprenant en outre un dispositif source d'alimentation (32), connecté électriquement à ladite pluralité de structures d'électrode (16) électriquement polarisables et configuré pour leur appliquer une polarisation.

**9.** Appareil (80) selon la revendication 8, dans lequel ledit dispositif source d'alimentation (32) est configuré pour fournir des signaux déphasés à des éléments individuels de ladite pluralité d'électrodes.

**10.** Appareil (80) selon la revendication 9, dans lequel lesdits signaux déphasés sont choisis de manière à générer une force diélectrophorétique à onde progressive.

**11.** Appareil (80) selon la revendication 9, dans lequel lesdits signaux déphasés sont choisis de manière à générer une force diélectrophorétique classique.

**12.** Procédé de manipulation d'un objet présent dans un fluide par électrocinétique, comprenant les étapes consistant à mettre en contact le fluide avec l'appareil (80) selon l'une quelconque des revendications 1 à 11 et à appliquer une polarisation à ladite pluralité de structures d'électrode électriquement polarisables (16) de manière à générer un champ électrique non uniforme, en manipulant de la sorte l'objet.

**13.** Procédé selon la revendication 12, dans lequel ledit fluide est choisi dans le groupe constitué d'eau, d'une suspension de cellules bactériennes, d'un milieu de protéine, d'un milieu d'anticorps, d'un milieu d'acide nucléique, d'encre, de solution saline tamponnée au phosphate et d'un fluide biologique.

**14.** Procédé selon la revendication 12, dans lequel ledit objet est sélectionné dans le groupe constitué d'une cellule, d'un agrégat de cellules, d'un érythrocyte normal, d'un érythrocyte anormal, d'un globule rouge nucléé foetal, d'une cellule cancéreuse, d'une protéine, d'ADN, d'ARN, d'un anticorps, d'un antigène, d'un lipide, d'un assemblage de molécules, d'un virus, d'un plasmide, d'une bactérie, d'un protozoaire, d'un minéral, d'un cristal, d'une particule conductrice, d'une particule semi-conductrice, d'une particule diélectrique et d'une bulle de gaz.

10

20

14

18    16    18    18    16    18

12

## Fig. 1

10

30    34

38    36    40

32

42

## Fig. 2a

Fig. 2b

Fig. 3

**Fig. 4a**

Electric Field [AU]

**Fig. 4b**

$$\nabla E^2 / |\nabla E^2| \text{ [AU]}$$

Fig. 4c

Fig. 5a

Fig. 5b

Fig. 5c

Fig. 5d

Fig. 6a

Fig. 6b

**Fig. 6c**

**Fig. 6d**

Fig. 6e

Fig. 6f

**Fig. 6g**

Electric Field [AU]

Height [d]

Axial Location [d]

**Fig. 6h**

Electric Field [AU]

Height [d]

Axial Location [d]

Fig. 6i

$\nabla E^2 / |\nabla E^2|$ [AU]

Fig. 6j

$\nabla E^2 / |\nabla E^2|$ [AU]

Fig. 7a

Electric Field [AU]

Fig. 7b

∇E² [AU]

**Fig. 7c**

Electric Field [AU]

**Fig. 7d**

∇E² [AU]

**Non Dimensional Potential**

Fig. 8a

**Non Dimensional Potential * 1000**

Fig. 8b

**Electric Field [AU]**

Fig. 8c

Fig. 8d

Fig. 8e

Fig. 8f

Fig. 8g

Fig. 8h

Fig. 9

Fig. 10a

Fig. 10b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040011650 A
- EP 1548103 A
- WO 0028313 A
- WO 2006004558 A

**Non-patent literature cited in the description**

- **GREEN, N. G. ; MORGAN, H.** *J. Phys. D,* 1998, vol. 31, L25-L30
- **MORGAN, H. ; HUGHES, M. P. ; GREEN, N. G.** *Biophys. J.,* 1999, vol. 77, 516-525
- **LI, H. ; BASHIR, R.** *Sensors aszd Actuators,* 2002, vol. 86, 215-221
- **TALARY, M. S. ; BURT, J. P. H. ; TAME, J. A. ; PETHIG, R.** *J. Phys. D,* 1996, vol. 29, 2198-2203
- **XU, J. Q. ; WU, L. ; HUANG, M. ; YANG, W. ; CHENG, J. ; WANG, X.** *Micro Total Analysis Systems,* 2001, 565-566
- **WANG, X. ; YANG, J. ; HUANG, Y. ; VYKOUKAL, J. ; BECKER, F. F. ; GASCOYNE, P. R. C.** *Anal. Chem.,* 2000, vol. 72, 832-839